Europäisches Patentamt

European Patent Office

Office européen des brevets

(19)

(11) Veröffentlichungsnummer: **0 496 393 B1**

(12) **EUROPÄISCHE PATENTSCHRIFT**

(45) Veröffentlichungstag der Patentschrift: **25.01.95**

(51) Int. Cl.⁶: **C07C 281/06**, C07K 1/00

(21) Anmeldenummer: **92101078.1**

(22) Anmeldetag: **23.01.92**

(54) **Alkylierung von Azaglycin-Derivaten.**

(30) Priorität: **24.01.91 DE 4102015**

(43) Veröffentlichungstag der Anmeldung:
**29.07.92 Patentblatt 92/31**

(45) Bekanntmachung des Hinweises auf die
Patenterteilung:
**25.01.95 Patentblatt 95/04**

(84) Benannte Vertragsstaaten:
**AT BE CH DE DK ES FR GB GR IT LI LU NL PT SE**

(56) Entgegenhaltungen:

**TETRAHEDRON LETTERS Bd. 29, Nr. 37, 1988, Seiten4661 - 4664; T. KOLASA ET AL: 'Oxidations of some alpha-aminoacids underMitsunobu reaction conditions'**

(73) Patentinhaber: **HOECHST AKTIENGESELL-SCHAFT**

**D-65926 Frankfurt (DE)**

(72) Erfinder: **Talaga, Patrice, Dr.**
**Avenue Van Becelaere, 24A, boite 49**
**B-1170 Bruxelles (BE)**
Erfinder: **König, Wolfgang, Dr.**
**Eppsteiner Strasse 25**
**W-6238 Hofheim am Taunus (DE)**

**Beschreibung**

Das Redox Condensation System Diethylazodicarbonsäure (DEAD) - Triphenylphosphin (TPP), besser bekannt als das Mitsunobu System, ist eine sehr beliebte Methode um Alkylierungsreaktionen (beispielsweise an Imiden, heterocyclischen Strukturen usw.) durchzuführen (O. Mitsunobu, Synthesis 1981, 1). Primäre und sekundäre Amide können jedoch mit Hilfe dieses Systems nicht alkyliert werden (S. Bittner et al., J. Org. Chem. 1985, 50, 1712).

Überraschenderweise fanden wir, daß acylierte Azaglycin-Derivate mit Hilfe der Mitsunobu-Reaktion alkyliert werden können.

Gegenstand der Erfindung ist daher ein Verfahren zur Herstellung alkylierter Azaglycin Derivate der allgemeinen Formel I,

$$X\text{-}(A)_n\text{-}N(R)\text{-}NH\text{-}CO\text{-}NH_2, \qquad (I)$$

worin

- X für eine Aminoschutzgruppe, $C_1$-$C_8$-Alkanoyl, $C_6$-$C_{14}$-Arylcarbonyl oder $C_6$-$C_{14}$-Aryl-$C_1$-$C_4$-alkanoyl,
- A für gegebenenfalls an der Drittfunktion geschützte Aminosäure- oder Iminosäurereste,
- n 0 - 10, bevorzugt 1 - 5, wobei X $C_1$-$C_8$-Alkanoyl, $C_6$-$C_{14}$-Arylcarbonyl oder $C_6$-$C_{14}$-Aryl-$C_1$-$C_4$-alkanoyl bedeutet falls n = 0 ist, und
- R für $C_1$-$C_8$-Alkyl, $C_6$-$C_{14}$-Aryl-$C_1$-$C_4$-alkyl oder $C_5$-$C_{12}$-Heteroaryl-$C_1$-$C_4$-alkyl steht,

dadurch gekennzeichnet, daß man eine Verbindung der allgemeinen Formel II

$$X\text{-}(A)_n\text{-}NH\text{-}NH\text{-}CO\text{-}NH_2 \qquad (II),$$

in der X, A und n die obengenannten Bedeutungen haben, mit einem primären oder sekundären Alkohol und überschüssigem DEAD, bevorzugt ca. 3 Equivalenten, sowie einem Tri-$C_1$-$C_6$-alkylphosphin, Tri-$C_6$-$C_{14}$-arylphosphin oder Pyridyl-di-$C_6$-$C_{14}$ arylphosphin, wobei der Arylteil gegebenenfalls durch Di-$C_1$-$C_4$-alkylamino substituiert sein kann, in einem Ether bei 0 °C bis 30 °C umsetzt und die Aminoschutzgruppe X gegebenenfalls nach den allgemein bekannten Methoden abspaltet, mit der Maßgabe, daß bei der Verwendung von Tri-n-butyl-phosphin X nicht Fmoc bedeutet.

Unter den Phosphin-Derivaten werden bevorzugt Triphenylphosphin, Tri-n-butylphosphin, p-Dimethyl-aminophenyl-diphenylphosphin (M. von Itzstein et al., Synth. Comm. 1990, 20, 2049), Pyridyl-diphenylphosphin (Ibid), und polymergebundenes Triphenylphosphin (R.A. Amos, J. Org. Chem. 1983, 48, 3598), insbesondere Triphenylphosphin, verwendet.

Die zur Alkylierung verwendeten Alkohole sind vorzugsweise primäre und sekundäre $C_1$-$C_{18}$-Alkylalkanole, wie z.B. Methanol, Ethanol, $C_6$-$C_{14}$-Aryl-$C_1$-$C_4$-alkanole, wie z.B. Benzylalkohol, Naphthyl-$C_1$-$C_4$-alkanol, oder $C_5$-$C_7$-Heteroarylalkohole, wie z.B. Pyridyl-$C_1$-$C_4$-alkanol, wobei die Aryl- und Heteroarylalkohole im aromatischen Ring auch durch Halogen, wie Fluor, Chlor, Brom oder Jod, $C_1$-$C_6$-Alkyl oder $C_1$-$C_6$-Alkoxy substituiert sein können.

Eine Aminosäure wie vorstehend genannt sind alle natürlichen oder unnatürlichen Aminosäuren, die falls chiral, in der D- oder L-Form vorliegen. Bevorzugt sind $\alpha$-Aminosäuren. Beispielsweise seien genannt:

Aad, Abu, $\gamma$Abu, ABz, 2ABz, $\epsilon$Aca, Ach, Acp, Adpd, Ahb, Aib, $\beta$Aib, Ala, $\beta$Ala, $\Delta$Ala, Alg, All, Ama, Amt, Ape, Apm, Apr, Arg, Asn, Asp, Asu, Aze, Azi, Bai, Bph, Can, Cit, Cys, (Cys)$_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPro, hPhe, hSer, hThr, hTrp, hTyr, Hyl, Hyp, 3Hyp, Ile, Ise, Iva, Kyn, Lant, Lcn, Leu, Lsg, Lys, $\beta$Lys, $\Delta$Lys, Met, Mim, Min, nArg, Nle, Nva, Oly, Orn, Pan, Pec, Pen, Phe, Phg, Pic, Pro, $\Delta$Pro, Pse, Pya, Pyr, Pza, Qin, Ros, Sar, Sec, Sem, Ser, Thi, $\beta$Thi, Thr, Thy, Thx, Tly, Tia, Tle, Trp, Trta, Tyr, Val, Nal, Tbg, Npg, Chg, Cha, Thia (vgl. z.B. Houben-Weyl, Methoden der organischen Chemie, Band XV/1 und 2, Stuttgart 1974).

Unter einer Iminosäure werden allgemein natürliche oder unnatürliche Aminosäuren, deren Aminogruppe monosubstituiert ist, verstanden. Besonders seien in diesem Zusammenhang Verbindungen genannt, die durch $C_1$-$C_8$-Alkyl, das wiederum gegebenenfalls einfach oder zweifach ungesättigt ist und das durch bis zu drei gleiche oder verschiedene Reste aus der Reihe Mercapto; Hydroxy; $C_1$-$C_7$-Alkoxy; Carbamoyl; $C_1$-$C_8$-Alkanoyl; Carboxy; $C_1$-$C_7$-Alkoxycarbonyl; F; Cl; Br; I; Amino; Amidino, das gegebenenfalls durch einen, zwei oder drei $C_1$-$C_6$-Alkylreste substituiert sein kann; Guanidino, das gegebenenfalls durch einen oder zwei Benzyloxycarbonylreste oder durch eine, zwei, drei oder vier $C_1$-$C_8$-Alkylreste substituiert sein kann; $C_1$-$C_7$-Alkylamino; Di-$C_1$-$C_7$-Alkylamino; $C_1$-$C_6$-Alkoxycarbonylamino; $C_7$-$C_{15}$-Aralkoxycarbonyl; $C_7$-$C_{15}$-Aralkoxycarbonylamino; Phenyl-$C_1$-$C_4$-alkoxy; 9-Fluorenylmethoxycarbonylamino; $C_1$-$C_6$-Alkylsulfonyl; $C_1$-$C_6$-Alkylsulfinyl; $C_1$-$C_6$-Alkylthio; Hydroxamino; Hydroximino; Sulfamoyl; Sulfo; Carboxamido; Formyl; Hydrazono oder Imino substituiert sein kann. Ferner kommen Heterocyclen aus der folgenden Gruppe in Betracht:

Pyrrolidin-2-carbonsäure, Piperidin-2-carbonsäure, 1,2,3,4-Tetrahydroisochinolin-3-carbonsäure, Decahydroisochinolin-3-carbonsäure, Octahydroindol-2-carbonsäure, Decahydrochinolin-2-carbonsäure, Octahydrocyclopenta[b]pyrrol-2-carbonsäure, 2-Aza-bicyclo[2.2.2]octan-3-carbonsäure, 2-Azabicyclo[2.2.1]heptan-3-carbonsäure, 2-Azabicycol[3.1.0]hexan-3-carbonsäure, 2-Azaspiro-[4.4]nonan-3-carbonsäure, 2-Azaspiro[4.5]decan-3-carbonsäure, Spiro[(bicyclo[2.2.1]heptan)-2,3-pyrrolidin-5-carbonsäure], Spiro[(bicyclo[2.2.2]octan)-2,3-pyrrolidin-5-carbonsäure], 2-Azatricyclo[4.3.0.1$^{6,9}$]-decan-3-carbonsäure,Decahydrocyclohepta[b]-pyrrol-2-carbonsäure, Decahydrocycloocta[b]pyrrol-2-carbonsäure, Octahydroisoindol-1-carbonsäure, 2,3,3a,4,6a-Hexahydrocyclopenta[b]pyrrol-2-carbonsäure, 2,3,3a,4,5,7a-Hexahydroindol-2-car-bonsäure, Tetrahydrothiazol-4-carbonsäure, Isoxa-zolidin-3-carbonsäure, Pyrazolidin-3-carbonsäure, Hydroxyprolin-2-carbonsäure, die alle gegebenenfalls substituiert sein können.

Die oben genannten Resten zugrundeliegenden Heterocyclen sind beispielsweise bekannt aus US-A 4,344,949, US-A 4,374,847, US-A 4,350,704, EP-A 29 488, EP-A 31 741, EP-A 46 953, EP-A 49 605, EP-A 49 658, EP-A 50 800, EP-A 51 020, EP-A 52 870, EP-A 79 022, EP-A 84 164, EP-A 89 637, EP-A 90 341, EP-A 90 362, EP-A 105 102, EP-A 109 020, EP-A 111 873, EP-A 113 880, EP-A 271 865 und EP-A 344 682.

Unter einer Aminoschutzgruppe werden alle in der Peptidchemie üblichen Aminoschutzgruppen verstanden, wie z.B. Acetamidomethyl (Acm), 1-Adamantyloxycarbonyl (Adoc), 1-(1-Adamantyl)-1-methyl-ethoxycarbonyl (Adpoc), Allyloxycarbonyl (Aloc), tert.-Butyloxycarbonyl (Boc), 1-(4-Biphenylyl)-1-methyl-ethoxycarbonyl (Bpoc), $\alpha,\alpha$-Dimethyl-3,5-dimethoxybenzyloxycarbonyl (Ddz), 4-Dih-ydroxaborylbenzyloxycarbonyl (Dobz), 9-Fluorenyl-methyloxycarbonyl (Fmoc), Isobornyloxycarbonyl (Iboc), 1-Methyl-cyclobutyloxycarbonyl (Mboc), 4-Methoxybenzyloxycarbonyl (Moc), Methylsulfonyl-ethoxycarbonyl (Msc), 2-Phosphonioethoxycarbo-nyl (Peoc), Phenylsulfonylethoxycarbonyl (Pse), 4-Pyridylmethoxycarbonyl (Pyoc), 2,2,2-Trichloro-tert.-butyloxycarbonyl (Tcboc), Toluolsulfonylethox-ycarbonyl (Tse), Benzyloxycarbonyl (Z), halogen-substituiertes Benzyloxycarbonyl (Z(Hal$_n$)), 4-Nitro-benzyloxycarbonyl (Z(NO$_2$)) (siehe auch z.B. T.W. Greene, "Protective Groups in Organic Chemistry", New York, John Wiley & Sons, 1981; A. Hubbuch, Kontakte Merck 1979, Nr. 3, Seiten 14 - 23). Bevorzugt sind Fmoc, Boc und Z. Drittfunktionen der Amino- bzw. Iminosäuren können durch geeignete Schutzgruppen geschützt, wie beispielsweise in E.E. Büllesbach, Kontakte Merck 1980, Nr. 1, Seiten 23 - 35 beschrieben, oder glycosyliert sein (siehe z.B. EP-A 263 521 (HOE 86/F 253)). Insbesondere seien genannt: Arg(Tos), Arg(Mts), Arg-(Mtr), Arg(Pmc), Asp(OBzl), Asp(OtBu), Cys(4-MeBzl), Cys(Acm), Cys(StBu), Glu(OBzl), Glu-(OtBu), His(Tos), His(Fmoc), His(Dnp), His(Trt), Lys(Cl-2), Lys(Boc), Met(O), Ser(Bzl), Ser(tBu), Thr-(Bzl), Thr(tBu).

C$_6$-C$_{14}$-Aryl ist beispielsweise Phenyl, Naphthyl, Biphenylyl oder Fluorenyl; bevorzugt sind Phe-nyl und Naphthyl. Unter C$_5$-C$_{12}$-Heteroaryl werden mono-, bi- oder tricyclische Heteroaromaten verstanden, die keine saure -NH-Gruppe enthalten. Beispielsweise seien genannt: Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl oder $\beta$-Carbolinyl.

Die alkylierten Peptide können durch Kristallisation oder durch Kieselgel-Chromatographie gereinigt werden. Bei der Verwendung von Triphenylphosphin ist das bei der Reaktion entstehende Triphenylphosphinoxid oft schlecht abtrennbar. Dieses Problem tritt mit wasserlöslichen Triarylphosphinen - wie z.B p-Dimethyl-aminophenyl-diphenylphosphin - nicht auf. Das hier entstehende Phosphinoxid-Derivat läßt sich sehr leicht von lipophilen Verbindungen - z.B. geschützten alkylierten Peptiden - abtrennen, da es in saurem wäßrigem Milieu gut löslich ist (M. von Itzstein et al., Synth. Comm. 1990, 20, 2049). Triphenylphosphinoxid kann jedoch auch dadurch abgetrennt werden, daß man die Aminoschutzgruppe abspaltet und danach die wasserlöslichen Salze der entstehenden Peptide von dem lipophilen Triphenylphosphinoxid leicht durch Verteilung zwischen Wasser und Essigester oder Ether abtrennt. Verwendet man polymergebundenes Triphenylphosphin, so kann das gebildete Triphenylphosphinoxid-Derivat durch Filtration abgetrennt werden. Bei Verwendung von Tri-n-bu-tylphosphin (PBu$_3$) wurde festgestellt, daß dieses Reagenz nicht mit der Fmoc-Schufzgruppe kompatibel ist. Die genannte Amino-Schutzgruppe wird während der Reaktion zum Teil abgespalten, so daß in diesem Fall eine basenstabile Schutzgruppe (wie z.B. die Boc-Gruppe) verwendet werden sollte.

Ausgangsprodukte:

a) Fmoc-Phe-Azagly-NH$_2$ (C$_{25}$H$_{24}$N$_4$O$_4$, MG 444).

Zu einer Lösung von 15 g (38,76 mmol) Fmoc-Phe-OH in 70 ml DMF gibt man 5,43 g (1 äq). HOBt, 4,32 g (1 äq.) HCl-semicarbazid und 4,90 ml (1 äq.) NAM. Man kühlt auf 0 °C und gibt unter Rühren 8,14 g (1 äg.) DCC hinzu. Man rührt eine Stunde bei 0 °C und läßt die Lösung über Nacht stehen. Anschließend wird die Lösung filtriert und einrotiert. Der Rückstand wird zwischen AcO-Et/Wasser verteilt. Beim Ausschütteln fällt das Peptid aus. Der Niederschlag wird abgesaugt und reichlich mit Wasser und anschließend mit Ether gewaschen und im Hochvakuum getrocknet. Man erhält 13,10 g (29,50 mmol) Peptid. Die Mutterlauge (AcOEt Phase) wird wie üblich behandelt (Waschen mit gesättigter NaHCO$_3$-Lösung (2 mal), Wasser, KHSO$_4$-Lösung, Wasser, trocknen über Natriumsulfat). Die organische Phase wird einrotiert, der Niederschlag mit Ether zerrieben und abgenutscht. Anschließend wird im Hochvakuum getrocknet. Man erhält weitere 0,92 g (2 mmol) Peptid.

Ausbeute: 14,02 g (31,57 mmol) = 81 %

Schmp.: 193 °C

$[\alpha]_D^{23}$ : - 16,2° (c = 1 in AcOH)

b) Boc-Phe-Azagly-NH$_2$ (C$_{15}$H$_{22}$H$_4$O$_4$, MG 322)

Zu einer Lösung von 30 g (113,2 mmol) Boc-Phe-OH in 100 ml DMF gibt man 15,85 g (1 äq.) HOBt, 12,61 g (1 äq.) HCl-Semicarbazid und 14,33 ml (1 äq.) NAM. Man kühlt auf 0 °C und gibt unter Rühren 23,77g (1 äq.) DCC dazu. Man läßt eine weitere Stunde Rühren und übers Wochenende stehen. Danach wird die Lösung filtriert und einrotiert. Der Rückstand wird zwischen AcOEt und Wasser verteilt, die organische Phase nacheinander mit gesättigter NaHCO$_3$-Lösung (3 mal), Wasser, KHSO$_4$-Lösung (2 mal) und Wasser gewaschen. Nach dem Trocknen wird die Lösung einrotiert, der Rückstand mit Ether zerrieben, abgenutscht und im Hochvakuum getrocknet.

Ausbeute: 20,31 g (63 mmol) = 57%

$[\alpha]_D^{23}$ : + 2,2° (c = 1 in AcOH)

Beispiel 1

a) Fmoc-Phe-N(Me)-NH-CONH$_2$ (C$_{26}$H$_{26}$N$_4$O$_4$, MG 458)

Zu 50 ml absolutem THF, bei 0 °C, gibt man 2,5 g (5,63 mmol) Fmoc-Phe-Azagly-NH$_2$ als Suspension, 1,83 ml (8 äq.) MeOH, 2,96 g (2 äq) Triphenylphosphin (PPh$_3$), portionsweise 1,773 ml (2 äq.) Diethylazodicarbonsäure (DEAD) und nach 15 min. Rühren weitere 738 mg (0,5 äq.) PPh$_3$ und 0,443 ml (0,5 äq.) DEAD zu. PPh$_3$ und DEAD werden nach weiteren 15 min. nochmals in gleicher Menge zugesetzt. Dann stellt man diese Suspension auf RT und läßt 4 Stunden rühren (kein Ausgangsmaterial mehr!). Diese Suspension wird filtriert, der Niederschlag mit Ether gewaschen und im Hochvakuum getrocknet. Man erhält 930 mg (2,03 mmol) reines N-Me Derivat (36 %). Anschließend wird die Mutterlauge einrotiert mit Ether zerrieben und abgesaugt. Dieser Niederschlag enthält die gewünschte Verbindung und Triphenylphosphinoxid (POPh$_3$).

Schmp: 173-174 °C

$[\alpha]_D^{23}$ = + 27° (c = 1 in AcOH)

b) HCl-Phe-N(Me)-NH-CONH$_2$ (C$_{11}$H$_{16}$N$_4$O$_2$, MG 236)

900 mg (1,96 mmol) reines Fmoc-Phe-N(Me)-NH-CONH$_2$ werden in 15 ml DMF gelöst. Dann gibt man 2,16 ml (10 äq.) Et$_2$NH dazu. Man rührt während 15 min. und rotiert die Lösung ein. Der Rückstand wird mit Ether zerrieben und abgenutscht. Anschließend wird die Verbindung in MeOH gelöst, mit 1N HCl auf pH 6,40 gebracht und die Lösung einrotiert. Der Rückstand wird mit Ether zerrieben, abgenutscht und im Hochvakuum getrocknet.

Ausbeute: 430 mg (1,58 mmol) = 80 %

Schmp: 98-105 °C

$[\alpha]_D^{23}$ = + 23 ° (c = 1 in AcOH)

Beispiel 2

a) Fmoc-Phe-N(Et)-NH-CONH$_2$ (C$_{27}$H$_{28}$N$_4$O$_4$, MG 472)

Zu 50 ml absolutem THF gibt man 4 g (9mmol) Fmoc-Phe-Azagly-NH$_2$ als Suspension, 4,23 ml (8 äq.) EtOH, 4,72 g (2 äq.) PPh$_3$ und 2,834 ml (2 äq.) DEAD. Innerhalb 15 min. werden noch zweimal 0,5 äq. PPh$_3$ (1,18 g) und DEAD (0,708 ml) zugegeben. Nach der zweiten Zugabe läßt man die Suspension 4 Stunden rühren. Dann wird die Lösung einrotiert und die Verbindung durch Kieselgel-Chromatographie gereinigt (Laufmittel: CH$_2$Cl$_2$/AcOEt = 9/1 und CH$_2$Cl$_2$/MeOH = 9,5/0,5). Die Fraktionen (mit POPh$_3$ verunreinigt) werden einrotiert und der Rückstand mit Petrolether (PE) zerrieben, abgenutcht und im Hochvakuum getrocknet.

b) HCl-Phe-N(Et)-NH-CONH$_2$ (C$_{12}$H$_{18}$N$_4$O$_2$, MG 250)

9 mmol des oben gewonnenen Peptids (mit POPh$_3$; gerechnet als 100 % Ausbeute) wird in 20 ml DMF gelöst. Nach Zugabe von 9,90 ml (10 äq.) Et$_2$NH läßt man 30 min. rühren und rotiert die Lösung ein. Der Rückstand wird zweimal mit PE zerrieben, anschließend in einer Mischung AcOEt/Wasser suspendiert und unter Rühren mit 1N HCl auf pH 6,40 gebracht. Dann wird diese Mischung ausgeschüttelt, die Wasserphase 3 mal mit Ether gewaschen, etwas einrotiert (Rest Ether und AcOEt abdestilliert), filtriert und gefriergetrocknet.

Ausbeute: 1,42 g (5,68 mmol) = 56 %

Schmp: 91-94 °C

$[\alpha]_D^{23}$ = + 62,5 ° (c = 1 in AcOH).

Beispiel 3

a) Fmoc-Phe-N(iPr)-NH-CONH$_2$ (C$_{28}$H$_{30}$N$_4$O$_4$, MG 486)

Die Titelverbindung wird analog Beispiel 2a hergestellt.
Reagenzien:
- Fmoc-Phe-Azagly-NH$_2$: 4 g, 9 mmol
- iPrOH: 5,50 ml (8 äq.)
- PPh$_3$: 4,72 g (2 äq.)
- DEAD: 2,834 ml (2 äq.)
- zweimal 1,18 g PPh$_3$ (0,5 äq.) und 0,708 ml DEAD (0,5 äq.)

Nach 4 Stunden wird die Lösung einrotiert und das Peptid durch Kieselgel-Chromatographie gereinigt (Laufmittel: CH$_2$Cl$_2$/AcOEt = 9/1 und CH$_2$Cl$_2$/MeOH = 9/1). Die Fraktionen (Peptide mit POPh$_3$) werden einrotiert, der Rückstand mit PE zerrieben, abgenutscht und im Hochvakuum getrocknet.

b) HCl-Phe-N(iPr)-NH-CONH$_2$ (C$_{13}$H$_{20}$N$_4$O$_2$, MG 264)

Ansatz von 9 mmol; analog Beispiel 2b.
Ausbeute: 1,48 g (5,60 mmol) = 55 %
Schmp: 105-110 °C

Beispiel 4

a) Boc-Phe-N(CH$_2$-(2-Pyridyl))-NH-CONH$_2$ - (C$_{21}$H$_{27}$N$_5$O$_4$, MG 413)

Zu 50 ml absolutem THF bei 0 °C werden 4 g (12,42 mmol) Boc-Phe-Azagly-NH$_2$ 4,80 ml (2 äq.) 2-Pyridyl-methanol, 6,51 g (2 äq.) PPh$_3$, 3,911 ml (2 äq.) DEAD und innerhalb von 15 min. noch zweimal 1,63 g (0,5 äq.) PPh$_3$ und 0,978 ml (0,5 äq.) DEAD gegeben. Man rührt die Lösung zwei Stunden bei 0 °C und zwei Stunden bei RT. Dann wird die Lösung einrotiert, der Rückstand zwischen Ether und Wasser verteilt und schnell abdekantiert. Die organische Phase wird 30 min. bei 0 °C stehen gelassen. Dann wird der Niederschlag abgenutscht, mit kaltem Ether gewaschen und im Hochvakuum getrocknet. Man erhält 1,41 g (3,41 mmol) reine Verbindung. Die Mutterlauge wird einrotiert und mit Wasser zerrieben bis man eine feste Substanz bekommt. Dann wird dieser Niederschlag in CH$_2$Cl$_2$ aufgenommen, getrocknet und einrotiert. Diese Fraktion wird durch Kieselgel-Chromatographie (Laufmittel CH$_2$Cl$_2$/MeOH = 9/0,4, dann CH$_2$Cl$_2$/MeOH = 9/1) gereinigt. Man erhält 370 mg (0,89 mmol) reine Substanz.
Ausbeute: 1,78 g = 35 %
$[\alpha]_D^{23}$ : + 8,9 ° (c = 1 in AcOH)

b) 2 HCl-Phe-N(CH$_2$-(2-Pyridyl))-NH-CONH$_2$ - (C$_{16}$H$_{19}$N$_5$O$_2$, MG 313)

1,69 g (4,09 mmol) reines Boc-Phe-N(CH$_2$-(2-Pyridyl))-NH-CONH$_2$ werden in 20 ml Dioxan suspendiert. Bei 0 °C gibt man 20 ml gesättigte HCl-Dioxan Lösung dazu und läßt eine Stunde bei 0 °C rühren. Die Lösung wird dann einrotiert, der Rückstand zwischen AcOEt und Wasser verteilt und die Wasserphase noch zweimal mit AcOEt gewaschen. Dann wird die Wasserphase etwas einrotiert, filtriert und gefriergetrocknet.
Ausbeute: 1,29 g (3,34 mmol) = 84 %

Beispiel 5

a) Boc-Phe-N(CH$_2$-(2-Naphthyl))-NH-CONH$_2$ - (C$_{26}$H$_{30}$N$_4$O$_4$, MG 462)

Zu 50 ml absolutem THF bei 0 °C werden 4 g (12,42 mmol) Boc-Phe-Azagly-NH$_2$, 2,17 g (1,1 äq.) 2-Naphthalenmethanol, 6.19 ml (2 äq.) PBu$_3$ und 3,911 ml (2 äq.) DEAD gegeben. Man rührt diese Lösung 2 Stunden bei 0 °C und weitere 2 Stunden bei RT. Anschließend wird die Lösung einrotiert, der Rückstand mit 100 ml PE zerrieben und diese Mischung bei 0 °C 5 min. gerührt. Man fügt 10 bis 20 ml Ether hinzu und erhält dann einen weißen Niederschlag der abgenutscht wird. Es wird im Hochvakuum getrocknet. Man erhält 3,14 g Peptid-Derivat (mit POBu$_3$ verunreinigt). Die Mutterlauge wird einrotiert und in PE übers Wochenende im Kühlraum stehen gelassen. Dann wird der Niederschlag abgesaugt, mit kaltem PE gewaschen und im Hochvakuum getrocknet. Man erhält noch 1,40 g Rohpeptid (Peptid mit POBu$_3$). Diese zweite Fraktion wird anschließend durch Kieselgel-Chromatographie gereinigt (Laufmittel: CH$_2$Cl$_2$/MeOH = 9/0,5). Die Fraktionen werden einrotiert, der Rückstand mit PE zerrieben, abgenutscht und im Hochvakuum getrocknet.
Ausbeute: 520 mg
$[\alpha]_D^{23}$ = + 40,1 ° (c = 1 in AcOH)

b) HCl-Phe-N(CH$_2$(2-Naphthyl))-NH-CONH$_2$ - (C$_{21}$H$_{22}$N$_4$O$_2$, MG 362)

500 mg (1,08 mmol) reines Boc-Phe-N(CH$_2$(2-Naphthyl))-NH-CONH$_2$ werden in 10 ml Dioxan gelöst. Bei 0 °C gibt man unter Rühren 10 ml einer gesättigten HCl-Dioxan Lösung hinzu und rührt 1 Stunde bei 0 °C weiter. Dann wird die Lösung einrotiert, der Rückstand mit Ether zerrieben, abgenutscht und im Hochvakuum getrocknet.
Ausbeute: 360 mg (0,90 mmol) = 90 %

Beispiel 6

a) Z-Tyr(OiPr)-Pro-N(iPr)-NH-CONH$_2$ (C$_{29}$N$_{39}$N$_5$O$_6$, MG 553)

Die Titelverbindung wird analog Beispiel 2a hergestellt.
Reagenzien:
- Z-Tyr-Pro-Azagly-NH$_2$: 5 g (10 mmol)
- iPrOH: 5,80 ml (7,5 äq.)
- PPh$_3$: 5,25 g (2 äq.)
- DEAD: 3,15 ml (2 äq.)
- zweimal 1,31 g (0,5 äq.) PPh$_3$ und 0,80 ml (0,5 äq.) DEAD

Nach 4 Stunden wird die Lösung einrotiert und der Rückstand durch Kieselgel-Chromatographie gereinigt (Laufmittel: 1) CH$_2$Cl$_2$/Aceton = 9/1 und 2) CH$_2$Cl$_2$/MeOH = 9,5/0,5). Die Fraktionen werden einrotiert, der Rückstand mit PE zerrieben, abgenutscht und im Hochvakuum getrocknet.
Ausbeute: 4,46 g (8,06 mmol) = 80 %
$[\alpha]_D^{23}$ : - 23,5° (c = 1 in AcOH)

Verzeichnis der verwendeten Abkürzungen:

| | |
|---|---|
| Cha | Cyclohexylalanin |
| Chg | Cyclohexylglycin |
| DCC | Dicyclohexylcarbodiimid |
| DMF | Dimethylformamid |
| Nal | 3-(2-Naphthyl)alanin |
| NAM | N-Acetyl-morpholin |
| Npg | Neopentylglycin |
| PE | Petrolether |
| Tbg | Tert.-butylglycin |
| Thia | 2-Thienylalanin |

## Patentansprüche

1. Verfahren zur Herstellung alkylierter Azaglycin-Derivate der allgemeinen Formel I,

X-(A)$_n$-N(R)-NH-CO-NH$_2$,    (I)

worin
- X    für eine Aminoschutzgruppe, C$_1$-C$_8$-Alkanoyl, C$_6$-C$_{14}$-Arylcarbonyl oder C$_6$-C$_{14}$-Aryl-C$_1$-C$_4$-alkanoyl,
- A    für gegebenenfalls an der Drittfunktion geschützte Aminosäure- oder Iminosäurereste,
- n    0 - 10, wobei X C$_1$-C$_8$-Alkanoyl, C$_6$-C$_{14}$-Arylcarbonyl oder C$_6$-C$_{14}$-Aryl-C$_1$-C$_4$-alkanoyl bedeutet falls n = 0 ist, und
- R    für C$_1$-C$_8$-Alkyl, C$_6$-C$_{14}$-Aryl-C$_1$-C$_4$-alkyl oder C$_5$-C$_{12}$-Heteroaryl-C$_1$-C$_4$-alkyl steht,

dadurch gekennzeichnet, daß man eine Verbindung der allgemeinen Formel II,

X-(A)$_n$-NH-NH-CO-NH$_2$    (II),

in der X, A und n die obengenannten Bedeutungen haben, mit einem primären oder sekundären Alkohol und überschüssigem DEAD sowie einem Tri-C$_1$-C$_6$-alkylphospin, Tri-C$_6$-C$_{14}$-arylphosphin oder Pyridyl-di-C$_6$-C$_{14}$arylphosphin, wobei der Arylteil gegebenenfalls durch Di-C$_1$-C$_4$-alkylamino substituiert sein kann, in einem Ether bei 0 °C bis 30 °C umsetzt und die Aminoschutzgruppe X gegebenenfalls abspaltet, mit der Maßgabe, daß bei der Verwendung von Tri-n-butylphosphin X nicht Fmoc bedeutet.

2. Verfahren gemäß Anspruch 1, dadurch gekennzeichnet, daß Triphenylphosphin, Tri-n-butyl-phosphin, p-Dimethyl-aminophenyl-diphenylphosphin Pyridyl-diphenylphosphin oder polymergebundenes Triphenylphosphin verwendet werden.

## Claims

1. A process for the preparation of an alkylated azaglycine derivative of the formula I

X-(A)$_n$-N(R)-NH-CO-NH$_2$,    (I)

in which
- X    is an amino protective group, C$_1$-C$_8$-alkanoyl, C$_6$-C$_{14}$-arylcarbonyl or C$_6$-C$_{14}$-aryl-C$_1$-C$_4$-alkanoyl,
- A    is amino acid or imino acid radicals optionally protected on the third function,
- n    is 0-10, X being C$_1$-C$_8$-alkanoyl, C$_6$-C$_{14}$-arylcarbonyl or C$_6$-C$_{14}$-aryl-C$_1$-C$_4$-alkanoyl if n = 0, and
- R    is C$_1$-C$_8$-alkyl, C$_6$-C$_{14}$-aryl-C$_1$-C$_4$-alkyl or C$_5$-C$_{12}$-heteroaryl-C$_1$-C$_4$-alkyl,

which comprises reacting a compound of the formula II

X-(A)$_n$-NH-NH-CO-NH$_2$,    (II)

in which X, A and n have the abovementioned meanings, with a primary or secondary alcohol and excess DEAD, and a tri-C$_1$-C$_6$-alkyl-phosphine, tri-C$_6$-C$_{14}$-arylphosphine or pyridyl-di-C$_6$-C$_{14}$-arylphosphine, it being possible for the aryl moiety to be optionally substituted by di-C$_1$-C$_4$-alkylamino, in an ether at 0 °C to 30 °C and optionally removing the amino protective group X, with the proviso that X is not Fmoc when tri-n-butylphosphine is used.

**2.** The process as claimed in claim 1, wherein triphenylphosphine, tri-n-butylphosphine, p-dimethylaminophenyldiphenylphosphine, pyridyldiphenylphosphine or polymer-bound triphenylphosphine is used.

**Revendications**

**1.** Procédé pour la préparation de dérivés alkylés d'azaglycine, de formule générale I

$$X\text{-}(A)_n\text{-}N(R)\text{-}NH\text{-}CO\text{-}NH_2 \qquad (I)$$

dans laquelle

X   représente un groupe protecteur de fonction amino, un groupe alcanoyle en $C_1$-$C_8$, aryl($C_6$-$C_{14}$)-carbonyle ou aryl($C_6$-$C_{14}$)-alcanoyle($C_1$-$C_4$),

A   représente des restes d'aminoacides ou d'iminoacides éventuellement protégés sur la troisième fonction,

n   va de 0 à 10, lorsque n = 0 X représentant un groupe alcanoyle en $C_1$-$C_8$, aryl($C_6$-$C_{14}$)-carbonyle ou aryl($C_6$-$C_{14}$)-alcanoyle($C_1$-$C_4$), et

R   représente un groupe alkyle en $C_1$-$C_8$, aryl($C_6$-$C_{14}$)-alkyle-($C_1$-$C_4$) ou hétéroaryl($C_5$-$C_{12}$)-alkyle($C_1$-$C_4$),

caractérisé en ce que l'on fait réagir dans un éther, à 0-30°C, un composé de formule générale II

$$X\text{-}(A)_n\text{-}NH\text{-}NH\text{-}CO\text{-}NH_2 \qquad (II)$$

dans laquelle X, A et n ont les significations données plus haut, avec un alcool primaire ou secondaire et du DEAD en excès, ainsi qu'avec une trialkyl($C_1$-$C_6$)-phosphine, triaryl-($C_6$-$C_{14}$)-phosphine ou pyridyl-diaryl($C_6$-$C_{14}$)-phosphine, le fragment aryle pouvant éventuellement être substitué par un groupe dialkyl($C_1$-$C_4$)-amino, et éventuellement on élimine le groupe X protecteur de fonction amino, étant entendu que lorsqu'on utilise la tri-n-butylphosphine, X ne représente pas le groupe Fmoc.

**2.** Procédé selon la revendication 1, caractérisé en ce que l'on utilise la triphénylphosphine, la tri-n-butylphosphine, la p-diméthylaminophényl-diphénylphosphine, la pyridyl-diphénylphosphine ou de la triphénylphosphine liée à un polymère.